# EUROPEAN PATENT SPECIFICATION

(11) **EP 0 399 271 B1**
(45) Date of publication and mention of the grant of the patent: **10.01.1996**
(21) Application number: 90108538.1
(22) Date of filing: 07.05.1990
(51) Int. Cl.: G01N 33/574

(54) **Method for the detection of disease**
Verfahren zum Nachweis von Krankheiten
Procédé pour detecter des maladies

(30) Priority: 08.05.1989 FI 892197
(43) Date of publication of application: 28.11.1990
(73) Proprietor: LOCUS GENEX OY, FI-00580 Helsinki (FI)
(72) Inventor: Partanen, Paul, FI-00980 Helsinki (FI); Ylätupa, Sari, FI-00960 Helsinki (FI); Paasivuo, Raili, FI-00140 Helsinki (FI); Virtanen, Ismo, FI-00390 Helsinki (FI)
(74) Representative: Dipl.-Phys.Dr. Manitz Dipl.-Ing. Finsterwald Dipl.-Ing. Grämkow Dipl.Chem.Dr. Heyn Dipl.Phys. Rotermund Morgan, B.Sc.(Phys.)

(56) References cited:
- EP-A- 0 344 134
- EP-A- 0 359 274
- DE-A- 3 743 402
- US-A- 4 894 326
- DATABASE WPIL, week 8833, Derwent Publications Ltd., London (GB); acc. no. 88-230677/
- JOURNAL OF CELL SCIENCE, vol. 88, 1987, Cambridge (GB); T. VARTIO et al., pp. 419-430/
- JOURNAL OF CELL BIOLOGY, vol. 108, no. 3, March 1989, New York, NY (US); B. CARNEMOLLA et al., pp. 1139-1148/
- ANNALES DE PATHOLOGIE, vol. 1, no. 2, 1981, Paris (FR); Ph. BIREMBAUTET et al., pp. 140-146/
- DIAGNOSTIC HISTOPATHOLOGY, vol. 4, 1981, Chichester (GB); J. LABAT-ROBERT et al., pp. 299-306/
- DATABASE WPIL, week 8414, Derwent Publications Ltd., London (GB); acc. no. 84-084467/
- DATABASE WPIL, week 9017, Derwent Publications Ltd., London (GB); acc. no. 90-128252/

## Description

### Technical Field of the Invention

This invention relates to a method for detecting the presence of cancer or an autoimmune disease in human or animal and to a diagnostic kit for use in detection of the presence of cancer or an autoimmune disease.

### Background of the Invention

The diagnosis of, e.g., malignant tumors is conventionally based on tissue investigation, such as biopsy.

The general object of the present invention is to provide a method which is based on a general marker to be found in a blood sample.

### General Description of the Invention

Now it has been discovered that cellular fibronectin in blood can be used as a marker for cancers such as malignant tumors, and autoimmune diseases.

Cellular fibronectin is detected by immunological methods using specific antibodies to the extra-domain sequence.

The method is especially suitable for the detection of carcinomas.

### Detailed Description of the Invention

Fibronectins are adhesive glycoproteins that have variable primary structures due to cell-type specific slicing of messinger-RNA. Fibronectins can be divided into two major groups: plasma fibronectins (pFn) and cellular fibronectins (cFn). Cellular fibronectin differs from plasma fibronectin in having the so called extra domain (ED) sequence in the molecule. This ED-sequence has been called as ED1, EDA and EIIIA. Later the term EDA is used in here. Plasma form of the fibronectin molecule is primarily produced by hepatocytes, while cellular form is produced locally. However, plasma also contains small quantities of the cellular form.

Cellular fibronectin is largely confined to the endothelium of larger blood vessels, whereas this form of the fibronectin molecule is lacking in adult tissues.

Monoclonal antibodies to EDA containing fibronectins can be prepared by conventional methods (Vartio, T. et al., Differential expression of the ED sequence-containing form of cellular fibronectin in embryonic and adult human tissues, J. Cell Sci. 88:419-430 (1987); Virtanen, I. et al., Differential expression of the extra domain-containing form of cellular fibronectin in human placentas at different stages of maturation, Histochemistry 90:25-30 (1988) ). By using a monoclonal antibody, it has been shown that in adult tissues EDA containing cellular fibronectin is only present in larger endothelia and smooth muscle cells but it is present in the stromal tissues of all carcinomas (Laitinen, L. et al., Distribution of extra domain (ED)-containing form of cellular fibronectin in human solid tumors, Int. Congr. Acad. Pathology, Dublin 1988, Abstr. 144).

Monoclonal or polyclonal antibodies to EDA or its synthetic parts, i.e. synthetic peptides of the EDA sequence, the whole EDA or shorter or longer parts of it, can be used to detect EDA or EDA containing polypeptides in body fluids. Any suitable immunological methods can be used to detect the EDA. Such methods are, for example, enzyme immuno assays (EIA), radio immuno assays (RIA), fluorescence immuno assays (FIA), luminescence immuno assay (LIA), latex or other agglutination methods, particle counting immuno assays (PCIA), immunoblottings, immunoprecitation methods, or methods utilizing different kinds of biosensors.

In the determination especially the ratio of EDA containing cellular fibronectin to total fibronectin seems to be valuable. However, the measurement of the concentration of just the EDA containing fibronectin only is enough to demonstrate a cancer or autoimmune disease. The concentration of total fibronectin in human plasma is about 300(+) microg/ml, while the concentration of cellular fibronectin in normal human plasma is about 3(+) microg/ml.

The clinical value of the determination of increased cellular fibronectin during different cancers and autoimmune diseases in body fluids is significant, because there are no other good general markers for these diseases available.

### Test results

A solid phase EIA method was used to detect EDA containing cellular fibronectin in human sera, plasma or BAL fluid.

Monoclonal antibodies 52 DH1 and 52 BF12 were used, (see Vartio et al., ibid.). 52 DH1 reacts only with the extra domain of cellular fibronectin and 52 BF12 reacts with fibronectin. Affinity purified immunoglobulins (G-protein Sepharose®, Pharmacia-LKB Biotechnology, Uppsala, Sweden) were used to develop the solid phase enzyme immunoassay. Immunoglobulins were IgGl class.

The solid phase enzyme immunoassay was sandwich type, where the 52 DH1 affinity purified immunoglobulins were used to coat the solid phase support (MaxiSorp® 8 microwells, Nunc, Kamstrup, Denmark) and the 52 BF12 affinity purified immunoglobulins conjugated to horseradish peroxidase used as detection system, or vice versa. The conjugated polyclonal antibody to fibronectin can naturally be used instead of labelled monoclonal (BF12), or vice versa.

It was found beneficial to dilute the samples before incubation. Preferable dilution was 1:50 - 1:100, whereby relatively small cFn concentrations (0.02 microg/ml) could still be detected.

The samples were incubated for 1 hour at 37 °C (except in substrate incubation 30 min at room temperature). The wash solution was 0.02 M PBS pH 7.0 + 0.1 % Tween® 20. The wells were washed 5 times with 200 microl. wash solution.

Colon carcinoma and autoimmune diseased human sera were used to detect elevated levels of cellular fibronectin. Healthy blood donor sera (from Finnish Red Cross Transfusion Center, Helsinki, Finland) were used to evaluate the average concentration of EDA containing fibronectin in human sera.

In patients with colon carcinoma it was possible to demonstrate elevated concentrations of EDA containing fibronectin in blood circulation. 20 out of the 20 studied colon carcinoma patients sera were positive for cellular fibronectin showing at least two fold increase of the concentration in their sera compared to healthy blood donors. In most cases studied autoimmune patients sera were negative for cFn, but for instance the AMA patient's serum was showing high concentration of EDA containing fibronectin.

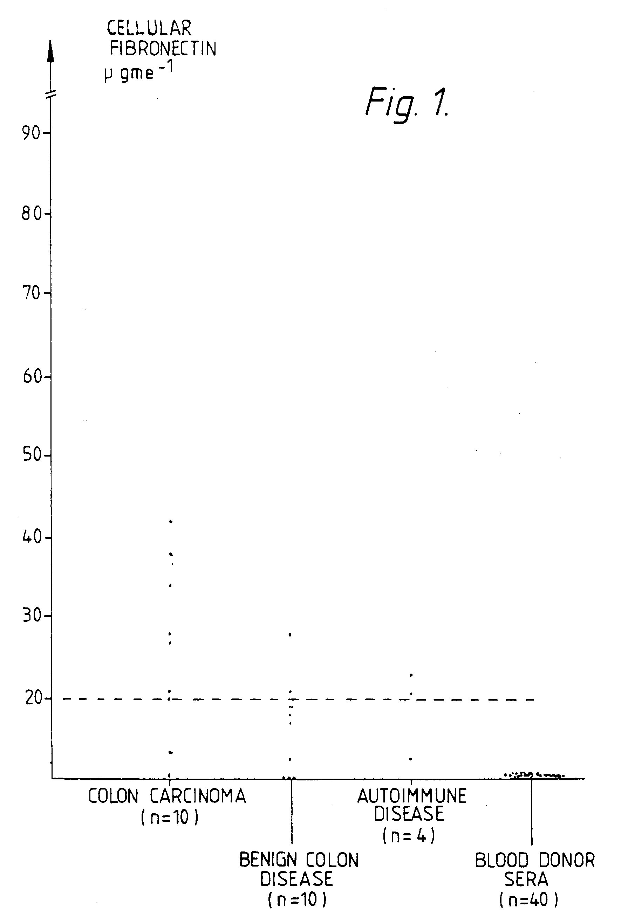

Results are illustrated in the enclosed Fig. 1.

## Claims

1. A method for detecting the presence of cancer or an autoimmune disease in human or animal, comprising determining the amount of cellular fibronectin by contacting an antibody specific for the extra domain sequence thereof with a blood sample, and using an elevated amount of cellular fibronectin as a marker for cancer or an autoimmune disease.

2. A method as claimed in claim 1, wherein the cancer is a malignant tumor.

3. A method as claimed in claim 2, wherein the tumor is carcinoma.

4. A method as claimed in claim 3, wherein the carcinoma is colon carcinoma.

5. A method as claimed in any one of the claims 1 to 4 wherein the sample is a serum sample.

6. A method as claimed in claim 1 wherein, additionally the amount of total fibronectin is determined, and the ratio of the amount of cellular fibronectin to the amount of total fibronectin is used as the marker.

7. Use of antibody specific for the extra domain sequence of cellular fibronectin for determining the amount of cellular fibronectin in a blood sample for detection of the presence of cancer or an autoimmune disease in human or animal.

## Patentansprüche

1. Verfahren zum Nachweis von Krebs oder einer Autoimmunkrankheit beim Menschen oder Tier, bei dem die Menge von Zellfibronectin bestimmt wird, indem man einen Antikörper, der für dessen Extradomainen-Sequenz spezifisch ist, mit einer Blutprobe in Kontakt bringt, und indem man eine erhöhte Menge des Zellfibronectins als einen Marker für Krebs oder eine Autoimmunkrankheit verwendet.

2. Verfahren nach Anspruch 1, bei dem der Krebs ein bösartiger Tumor ist.

3. Verfahren nach Anspruch 2, bei dem der Tumor ein Karzinom ist.

4. Verfahren nach Anspruch 3, bei dem das Karzinom ein Kolonkarzinom ist.

5. Verfahren nach einem der Ansprüche 1 bis 4, bei dem die Probe eine Serumprobe ist.

6. Verfahren nach Anspruch 1, bei dem zusätzlich die Menge des gesamten Fibronectins bestimmt wird und das Verhältnis der Menge des Zellfibronectins zu der Menge des gesamten Fibronectins als Marker verwendet wird.

7. Verwendung eines Antikörpers, der spezifisch für die Extradomainen-Sequenz des Zellfibronectins ist, für die Bestimmung der Menge von Zellfibronectin in einer Blutprobe, um Krebs oder eine Autoimmunkrankheit beim Menschen oder Tier nachzuweisen.

## Revendications

1. Procédé de détection de la présence du cancer ou d'une maladie auto-immune chez les êtres humains ou les animaux, qui consiste à déterminer la quantité de fibronectine cellulaire par mise en contact d'un anticorps spécifique pour la séquence de l'extra-domaine de celle-ci avec un échantillon de sang, et à utiliser une quantité élevée de fibronectine cellulaire comme marqueur pour le cancer ou une maladie auto-immune.

2. Procédé selon la revendication 1, dans lequel le cancer est une tumeur maligne.

3. Procédé selon la revendication 2, dans lequel la tumeur est un cancer.

4. Procédé selon la revendication 3, dans lequel le cancer est un cancer du colon.

5. Procédé selon l'une quelconque des revendications 1 à 4, dans lequel l'échantillon est un échantillon de sérum.

6. Procédé selon la revendication 1, dans lequel, en outre, la quantité de fibronectine totale est déterminée et le rapport de la quantité de fibronectine cellulaire à la quantité de fibronectine totale est utilisée comme marqueur.

7. Utilisation d'un anticorps spécifique pour la séquence de l'extra-domaine de la fibronectine cellulaire, pour déterminer la quantité de fibronectine cellulaire dans un échantillon sanguin, pour déterminer la présence du cancer ou d'une maladie auto-immune chez l'être humain ou l'animal.
